# EUROPEAN PATENT APPLICATION

(11) **EP 1 407 723 A2**
(43) Date of publication of application: **14.04.2004**
(21) Application number: 03425651.1
(22) Date of filing: 06.10.2003
(51) Int. Cl.: A61C 19/00

(54) **A dental unit**

(30) Priority: 08.10.2002 IT BO20020635
(71) Applicant: CASTELLINI S.p.A., I-40013 Castel Maggiore (Bologna) (IT)
(72) Inventor: Castellini, Franco, 40124 Bologna (IT)
(74) Representative: Lanzoni, Luciano

(57) **Abstract**

A dental unit (R) comprises: a base (1) supporting a plurality of dental handpieces; an arm (2) supporting a light source (3); a microprocessor unit (4) for the control of the components of dental unit (R); a first image display unit (5), comprising an intra-oral filming handpiece connected to the microprocessor unit (4) attached to a monitor (6) for display of the images, and a second image display unit (7) comprising equipment for filming the area around the dental unit (R) and connected, in turn, to the microprocessor unit (4).

## Description

The present invention relates to a dental unit in particular a dental unit equipped with video filming units which can be used during medical-surgical procedures in the odontotherapy and odontostomatology sector and also for environmental control use.

The current structuring of dentists' consulting rooms and the continuous improvement in the main and auxiliary equipment used on dental units, in addition to the ever higher level of professionalism of dentists, have led to an increase in the type of accessories that can be fitted on dental units, in particular of the most technologically advanced means in a number of sectors, such as information technology, digital video technology, etc.; this, for example, makes it possible to carry out particularly complex dental surgery procedures directly in the dentist's consulting rooms.

One example is the application and use, on dental units, of a camera, in the form of a handpiece for intra-oral filming, with which it is possible to view and record, thanks to a microprocessor unit, the oral cavity of the patient being treated and to observe it by means of an external monitor or one fitted directly to the dental unit.

At present, however, a much felt requirement concerns the possibility of viewing complex operations so as to allow the assistant to have a detailed and complete view of the area involved in the operation and in a comfortable position as well as not interfering with the movements of the dentist: a possibility which is not feasible with an intra-oral microcamera.

This situation would also make it possible to verify the outcome of the operation, the general post-operative situation of the area involved and of the surrounding areas and would also allow the operations to be recorded for archive and teaching purposes. This is also a requirement which is much felt in dentists' practices or universities where dentistry courses are held and where it is therefore necessary to back-up the lesions (or live operations) with video material.

Another requirement encountered in dentists' consulting rooms with more than one work station is being able to have the area around the dental unit under control, also from a remote work station, to check the use of the dental unit and, thus, to programme any disinfection-sterilisation cycles of the unit or, in any case, to allow environmental control of the area.

In line with the philosophy of continuous perfecting and technological completion of dental equipment, above all of dental units in general, the Applicant decided to equip the dental unit with an additional video unit designed to allow filming of the area around the dental unit in order to implement the possibilities of checking and recording the operative phases for constant and complete visual control of the area involved and for environmental control of the area around the dental unit.

The aim is achieved by means of a dental unit comprising: a base supporting a plurality of dental handpieces; an arm supporting a light source; a microprocessor unit which controls the components of the dental unit; a first image display unit, comprising an intra-oral filming handpiece connected to the microprocessor unit attached to a screen for the display of the images, and a second image display unit comprising equipment for filming the area around the dental unit and connected, in turn, to the microprocessor unit.

The technical characteristics of the invention, with reference to the above aims, are clearly described in the claims below and its advantages are apparent from the detailed description which follows, with reference to the accompanying drawings which illustrate a preferred embodiment of the invention provided merely by way of example without restricting the scope of the inventive concept, and in which:
- Figure 1 shows a dental unit according to the present invention;
- Figure 2 is a schematic side view of the dental unit according to the present invention, with some parts removed to better show others;

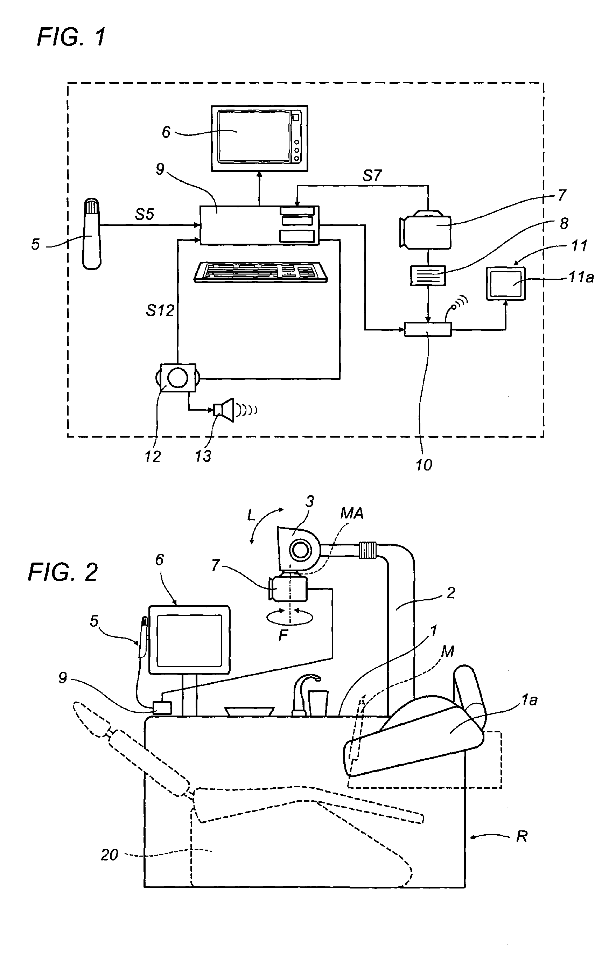

With reference to the accompanying drawings, in particular Figure 2, the dental unit R according to the present invention essentially comprises:
- a base 1 supporting a plurality of dental handpieces M;
- an arm 2 supporting a light source 3;
- a microprocessor unit 4 for the control of the components of the dental unit R;
- a first image display unit 5, comprising an intra-oral filming handpiece connected to the microprocessor unit 4 and attached to a monitor 6 for displaying the images;
- a second image display unit 7 comprising equipment for filming the area around the dental unit R and connected, in turn, to the microprocessor unit 4.

Obviously the dental unit R usually also consists of other parts, such as a chair 20 (shown with a dashed line) and a tray 1a forming the operative part of the base 1 supporting the handpieces M (shown here with a dashed line), but in this invention they are not particularly pertinent and will not be described in detail.

More specifically, the second display unit 7 preferably consists of a camera 7 which can be fitted, preferably but not restrictively, close to the light source 3 and therefore move together with this.

As can be seen in Figure 2, this light source consists of a lamp 3 supported by the arm 2 and the camera 7 can be associated with the lower part of the lamp. This configuration makes it possible to carry out a dual adjustment of the position of the camera 7 in order to choose the area around the dental unit 4 to be filmed.

The traditional adjustment movement of the lamp 3 (indicated with the arrow L) can in fact be combined with an adjustment movement of the camera 7 (see arrow F) around another axis perpendicular to the previous one; this movement can be controlled manually or power-driven by an independent drive schematically indicated with MA.

The camera 7 can also be equipped-associated with relative magnetic-optical-digital memory banks 8 for recording the images filmed by the camera.

According to what is shown in Figure 1, in the dental unit R in question, the microprocessor unit 4 is part of a computer unit (or simply of a PC) 9 positioned on the dental unit R itself.

The computer unit 9 can be directly interfaced, that is to say connected, with both the first and the second image display units 5 and 7 and with the monitor 6. In this case, the camera 7 can send the images to the computer unit and, preferably, download the images into recording memory banks 8a present in the computer unit 9, for the storage of the images.

The possibility of interfacing the computer unit 9 with the first and the second image display units 5 and 7 and with the monitor 6 allows both image signals S5 and S7 received from the first and second units 5 and 7 to be displayed on the same monitor 6.

Similarly, the computer unit 9 can allow the image signals S5 and S7 received from the first and second units 5 and 7 to be shown alternately on the monitor 6.

According to what is shown in Figure 1, the image display camera 7 (like the unit 5) can be connected directly, or by means of the interface of the computer unit 9, to a unit 10 which transmits the images to a remote unit 11 (for example by means of a modem, fibre optic cables, etc.) such as another monitor 11a.

Another possible addition is connection of the computer unit 9 to presence sensor means 12 in order to allow activation of the second display unit 7 on activation of a signal S12, and activation of units 13 sending alarm signals (where necessary).

Such a dental unit thus achieves the preset aims thanks to the addition of several independent display units and the aid of a computer unit designed to allow interfacing of the relative components. The integration of the two units is important since the antirolling handpiece provides limited field display while the camera 7 allows recording of the events around the operative area, which may concern the work of the dentist and that of the assistant.

This structure also makes it possible to operate and control numerous situations around the dental unit, from recording of the operations, as mentioned above, to controlling the area around the dental unit from remote locations not visually checked by operators.

The invention described can be subject to modifications and variations without thereby departing from the scope of the inventive concept. Moreover, all the details of the invention may be substituted by technically equivalent elements

## Claims

1. A dental unit comprising:
- a base (1), supporting a plurality of dental handpieces;
- an arm (2) supporting a light source (3);
- a microprocessor unit (4) for the control of the components of the dental unit (R);
- a first image display unit (5), comprising an antirolling filming handpiece connected to the microprocessor unit (4) and a monitor (6) for the display of the images:
- a second image display unit (7), comprising equipment for filming images of the area around the dental unit (R) and connected, in turn, to the microprocessor unit (4).

2. The dental unit according to claim 1, **characterised in that** the second image display unit (7) comprises memory banks (8) for recording the images.

3. The dental unit according to claim 1, **characterised in that** the microprocessor unit (4) is a part of a computer unit (9) positioned on the dental unit (R) and interfaced, that is to say connected, with the first and second image display units (5, 7).

4. The dental unit according to claim 1, **characterised in that** the second image display unit (7) is connected to a computer unit (9), of which the microprocessor unit (4) is a part, equipped with memory banks (8) for recording the images.

5. The dental unit according to claim 1, **characterised in that** the second image display unit (7) consists of a camera.

6. The dental unit according to claims 1 and 5,
**characterised in that** the camera (7) is fitted close to the light source (3).

7. The dental unit according to claim 6, **characterised in that** the light source consists of a lamp (3) and the camera (7) is associated with the lamp (3).

8. The dental unit according to claim 1, **characterised in that** the second display unit (7) consists of a camera whose position can be adjusted in order to choose the surrounding area to be viewed.

9. The dental unit according to claim 1, **characterised in that** the second display unit (7) consists of a camera associated with a lamp (3) forming the light source, the position of the camera (7) being adjustable in order to choose the surrounding area to be viewed.

10. The dental unit according to claims 1 and 3, **characterised in that** the computer unit (9) forms an interface between the first and second display units (5, 7) and the monitor (6) to allow both image signals (S5, S7) received from the first and second units (5, 7) to be displayed on the monitor (6) .

11. The dental unit according to claims 1 and 3, **characterised in that** the computer unit (9) forms an interface between the first and second display units (5, 7) and the monitor (6) to allow alternation on the monitor (6) of the image signals (S5, S7) received from the first and second units (5, 7).

12. The dental unit according to claim 1, **characterised in that** at least the second image display unit (7) is connected to a unit (10) which transmits the images to a remote unit (11).

13. The dental unit according to claims 1 and 3, **characterised in that** the computer unit (9) is connected at least to presence sensor means (12) to allow activation of the second display unit (7) and of units (13) sending alarm signals.
